# EUROPEAN PATENT APPLICATION

(11) **EP 1 679 369 A2**
(43) Date of publication of application: **12.07.2006**
(21) Application number: 04792334.7
(22) Date of filing: 06.10.2004
(51) Int. Cl.: C12N 15/00, C12Q 1/68, C12M 1/00

(54) **METHOD OF EXTENDING SINGLE-STRANDED NUCLEIC ACID, SINGLE-STRANDED NUCLEIC ACID EXTENDING APPARATUS AND DNA CHIP**

(30) Priority: 06.10.2003 JP 2003346779
(71) Applicant: SONY CORPORATION, Tokyo 141-0001 (JP)
(72) Inventor: MATSUMOTO, Sayoko, c/o Sony Corporation, Tokyo 141-0001 (JP); MAMINE, Takayoshi, c/o Sony Corporation, Tokyo 141-0001 (JP); WASHIZU, Masao, c/o Sony Corporation, Tokyo 141-0001 (JP); KUROSAWA, Osamu, c/o Sony Corporation, Tokyo 141-0001 (JP)
(74) Representative: DeVile, Jonathan Mark
(86) International application number: PCT/JP2004/015094
(87) International publication number: WO 2005/033326

(57) **Abstract**

To verify an action of a high-frequency ac electric field on a single-stranded nucleic acid existing in an aqueous solution. This action is used to improve the efficiency of hybridization to which the single-stranded nucleic acid is subjected as a complementary strand. Provided are a method and system for stretching a single-stranded nucleic acid, which exists in a free form in pure water or an aqueous solution (R) of pH 5 to 11, or which exists in a form immobilized on one of surface (f) of an electrode (E) of opposing electrodes (E,E) arranged facing the aqueous solution (R) or in a form immobilized on surfaces (f) of both electrodes (E) of opposing electrodes (E, E), by causing a high-frequency ac electric field to act on the single-stranded nucleic acid.

## Description

### Technical Field

This invention relates to a technique for stretching a single-stranded nucleic acid, which exists in a random-coil or like form in an aqueous solution, under the action of a high-frequency electric field.

### Background Art

There is a technology relating to integrated bioassay plates holding thereon predetermined DNAs microarrayed by microarray techniques and generally called "DNA chips" or "DNA microarrays" (hereinafter collectively called "DNA chips"). As a variety or number of DNA oligosaccharides, cDNAs (complementary DNAs) or the like are integrated on a glass substrate or silicon substrate, this DNA chip technology is characterized in that it permits a comprehensive analysis of an intermolecular reaction such as hybridization. Therefore, DNA chips are used in gene mutation analyses, SNPs (single-base polymorphisms) analyses, gene expression frequency analyses, and the like, and have begun to find utility in a wide range of fields such as drug developments, clinical diagnoses, pharmacogenomics, forensic medicine, and other fields.

The above-described DNA chip technology has now begun to shift from technical developments with efforts concentrated on increases in the numbers of DNA for comprehensive analyses to technical developments with aims directed to improvements in the accuracy of such comprehensive analyses and the efficiency of reactions.

More specifically, in view of the increasing application of DNA chips to clinical diagnoses and the like, demands have begun to arise for higher sensitivity, quantitativeness and accuracy, shorter reaction time and the like rather than the number of DNA integrated on each substrate.

Japanese Patent Laid-open No. Hei 6-038768 (see claim 1 and elsewhere) discloses a technique for eliminating thermal fluctuations in a high-viscosity solution or under an electric field under the premise of its application to a method or system that treats DNA, RNA, its derivative, its fragments by an enzymatic reaction or chemical reaction. This technique is described to permit efficiently and accurately performing the treatment of high molecules of DNA such as the synthesis reaction of DNA strands. Specifically, this technique is intended to allow high molecules of DNA to undergo an enzymatic reaction or chemical reaction under the existence of an electric field.

Further, Japanese Patent Laid-open No. Hei 8-322568 (claim 1, Paragraph [0001], and elsewhere) discloses a DNA replication process, which is characterized in that in an annealing reaction step of binding a primer to a single-stranded template DNA and a synthesis reaction step of allowing a DNA stand to stretch from the primer, an electric field is applied to a reaction solution with materials, a synthase and the like contained for the purpose of the synthesis to bring the template DNA into a linear form. This technique is intended for use in the sequential analysis for the determination of the base sequence of DNA or in the PCR method for the amplification of a DNA sample, and therefore, has as a premise that the components required for the above-described object are contained in the reaction solution to which an electric field is to be applied.

Nonetheless, nothing is known about any possible action or effect of a high-frequency ac electric field or any other electric field on a single-stranded nucleic acid existing in an aqueous solution of pure water or the like which is absolutely free of any components for the synthesis of DNA, such as a nucleic acid material, an enzyme and a primer. Further, no verification has been made yet about any possible correlation between the efficiency of hybridization in an aqueous solution, which is retained in a reaction well of a small volume on an integrated plate such as a DNA chip for bioassay, and a high-frequency ac electric field.

A primary object of the present invention is, therefore, to verify an action of a high-frequency ac electric field on a single-stranded nucleic acid existing in an aqueous solution, which is absolutely free of any components for the synthesis of DNA, such as a nucleic acid material, an enzyme and a primer; and to make use of the above-described action for improving the efficiency of hybridization in which the single-stranded nucleic acid is used as a complementary strand.

### Disclosure of Invention

The present invention, firstly, provides a nucleic acid stretch method of stretching the following single-stranded nucleic acid (1) or (2) by causing an ac electric field of a high frequency to act on the single-stranded nucleic acid (1) or (2): (1) a single-stranded nucleic acid existing in a free form in pure water or an aqueous solution of pH 5 to 11, or (2) a single-stranded nucleic acid existing in a form immobilized on one or both of opposing electrodes arranged facing said aqueous solution; and also a nucleic acid stretch system making use of the stretch means.

The present invention also provides a DNA chip making use of a means for stretching a single-stranded nucleic acid, which exists in a free or immobilized form in an aqueous solution of pH 5 to 11, under an action of a high-frequency ac electric field applied to a reaction well with pure water or said aqueous solution of pH 5 to 11 retained therein or under an action of dielectrophoresis.

It is to be noted that the term "aqueous solution" as used herein means pure water or an aqueous solution of pH 6 to 11 which is absolutely free of a nucleic acid material, an enzyme and any other high molecular component, such as a primer, for the purpose of DNA synthesis. The aqueous solution functions as a liquid phase which can provide a place of hybridization between nucleic acids having complementary strands.

### Brief Description of Drawings

Fig. 1 is a diagram schematically illustrating a state that a single-stranded nucleic acid (1) having a high-order structure entangled in a random coil form, such as DNA, exists in a free form in an aqueous solution (R).
Fig. 2 is a diagram schematically illustrating a state that under an action of a high-frequency ac electric field, the single-stranded nucleic acid (1) has been caused to direct in a single direction along the electric field.
Fig. 3 is a diagram showing a state that a single-stranded nucleic acid (3) immobilized at the position of a terminal thereof on a surface (f) of an electrode (E) is taking a high-order structure in a random coil form in a state of an impression voltage of 0.
Fig. 4 is a diagram schematically illustrating a state that a single-stranded nucleic acid (4) immobilized on the electrode (E) has been stretched by an action of a high-frequency ac electric field.
Fig. 5 is a diagram showing a construction that the other electrode (e) arranged opposite the electrode (E) is formed with a smaller area.
Fig. 6 depicts one embodiment of a reaction detecting section equipped with a construction that permits arraying reaction detecting sections on a plate such as a DNA chip.
Fig. 7 is an external perspective view showing one embodiment of a DNA chip (10) with the same reaction detecting sections (6) arrayed thereon.
Fig. 8 is a microphotograph (a photograph as a substitute for a drawing) obtained by an observation in an experiment, and is a photograph of a single strand of DNA in a random coil form in an aqueous solution before an electric field was applied.
Fig. 9 is a microphotograph (a photograph as a substitute for a drawing) obtained by another observation in the experiment, and is a photograph of single strands of DNA stretched by the application of the electric field.

### Best Mode for Carrying out the Invention

Based on the accompanying drawings, a description will be made of embodiments which can suitably carry out the technique according to the present invention for the stretch of a single-stranded nucleic acid.

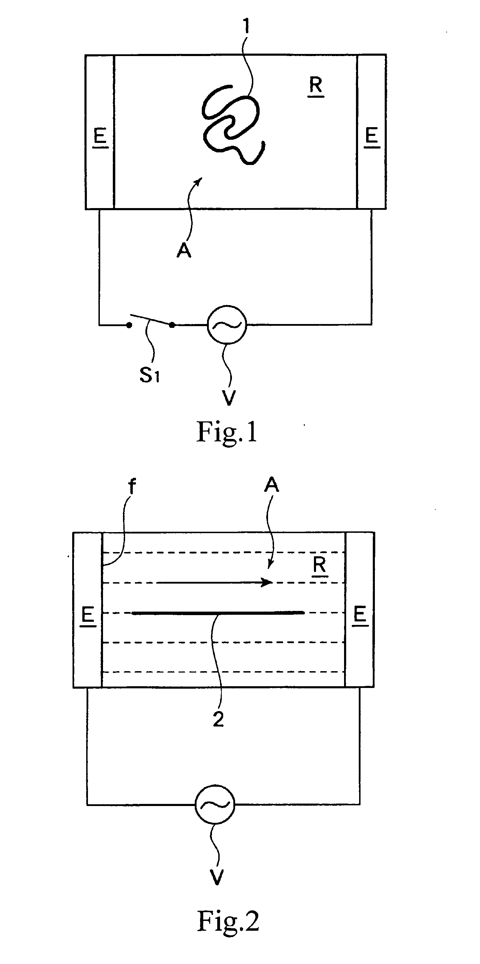

Fig. 1 schematically illustrates the state that a single-stranded nucleic acid 1 having a high-order structure entangled in a random coil form, such as DNA, exists in a free form in an aqueous solution indicated by sign R. In the state of Fig. 1, the applied voltage is 0. It is to be noted that in Fig. 1 and other drawings, sign A designates a reaction well capable of retaining the aqueous solution R and signs E,E indicate electrodes formed of aluminum or the like and arranged opposite to each other with the aqueous solution R, which is retained in the reaction well A, being sandwiched therebetween. Further, sign V, sign S₁ and sign S₂ indicate an ac power supply connected to the electrodes E,E, a switch in an OFF position, and a switch in an ON position, respectively.

It is desired to design the distance between the electrodes E-E at 40 µm or shorter, because a distance greater than 40 µm between the electrodes E-E is considered to readily induce convection in the aqueous solution R by thermal energy resulting from the application of a voltage. As this convection is considered to interfere with the action of a high-frequency ac electric field for the stretch of the single-stranded nucleic acid 1, it is desired to avoid, to the utmost, the occurrence of such convection in the aqueous solution R.

As illustrated in Fig. 2, a high-frequency ac electric field (which is indicated by dashed lines in the drawing) is formed in the aqueous solution R by the electrodes E,E across which a voltage is applied by the power supply V. By the action of this high-frequency ac electric field, it is possible to have the single-stranded nucleic acid 1 oriented in a single direction along the electric field in a state that no electrolysis takes place. The single-stranded nucleic acid is, therefore, illustrated in a form stretched as a result of the formation of the high-frequency ac electric field. It is to be noted that sign 2 designates the single-stranded nucleic acid which has been brought into the stretched form.

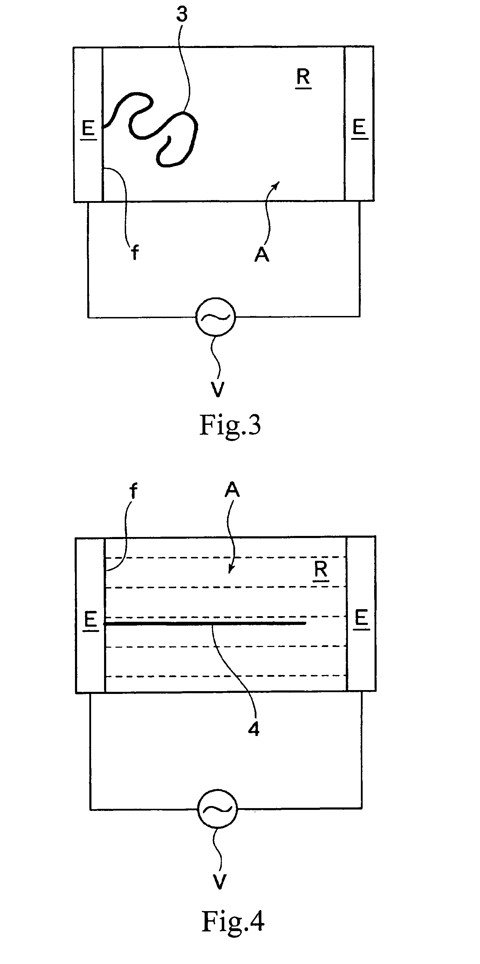

Fig. 3 shows the state that a single-stranded nucleic acid 3 immobilized at the position of the terminal thereof on the surface f of one of the electrodes E,E is taking a high-order structure in a random coil form in the state of an impression voltage of 0. It is to be noted that the electrode surface f has been surface-treated beforehand such that the terminal of the single-stranded nucleic acid 3 is immobilized there by chemical bonding such as coupling. On the surface f surface-treated with streptavidin, for example, a biotinylated terminal of a single-stranded nucleic acid can be immobilized.

Fig. 4 illustrates the state that an immobilized single-stranded nucleic acid 4 has been stretched by the action of a high-frequency ac electric field. The immobilized single-stranded nucleic acid 4 is caused to direct in a single direction while being immobilized, and as a result, is stretched along the electric field. It is to be noted that, even once the single-stranded nucleic acid has stretched, the single-stranded nucleic acid returns into the original random coil form when the application voltage for the high-frequency ac electric field is lowered to 0.

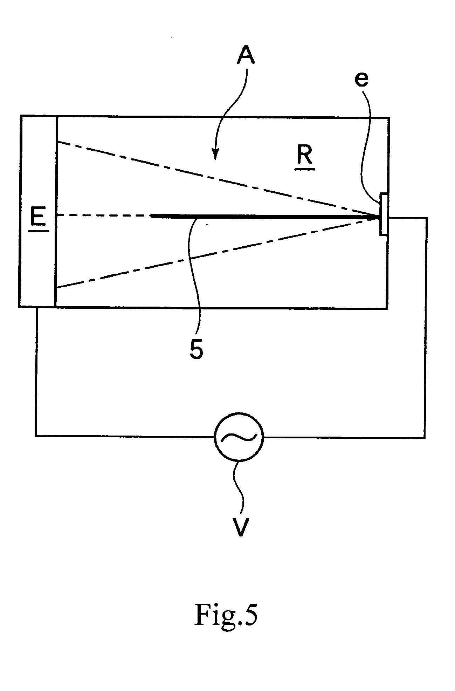

In the construction depicted in Fig. 5, the other electrode e arranged opposite the electrode E is formed with a smaller area so that a non-uniform electric field is formed concentrating on the electrode e (as indicated by alternate long-and-short lines in Fig. 5). It is to be noted that for the formation of a non-uniform electric field, a construction with the surfaces of electrodes formed into rough surfaces having concavities and convexities by surface treatment such as sputtering and by etching or the like or a like construction can be adopted, because electric lines of force concentrate at the convexities and pointed edge portions of the electrodes.

When contrived to form a non-uniform electric field in the vicinity of the electrode e such that electric lines of force concentrate at a single position, the single-stranded nucleic acid 1 existing in a free form in the aqueous solution R (see Fig. 1) can be caused to migrate by dielectrophoresis toward the single position (electrode e), at which the electric lines of force concentrate, while causing it to stretch. As a result, the single-stranded nucleic acid can be immobilized at the position of its terminal on the electrode e. It is to be noted that sign 5 in Fig. 5 indicates the single-stranded nucleic acid immobilized at the position of the terminal thereof on the electrode e.

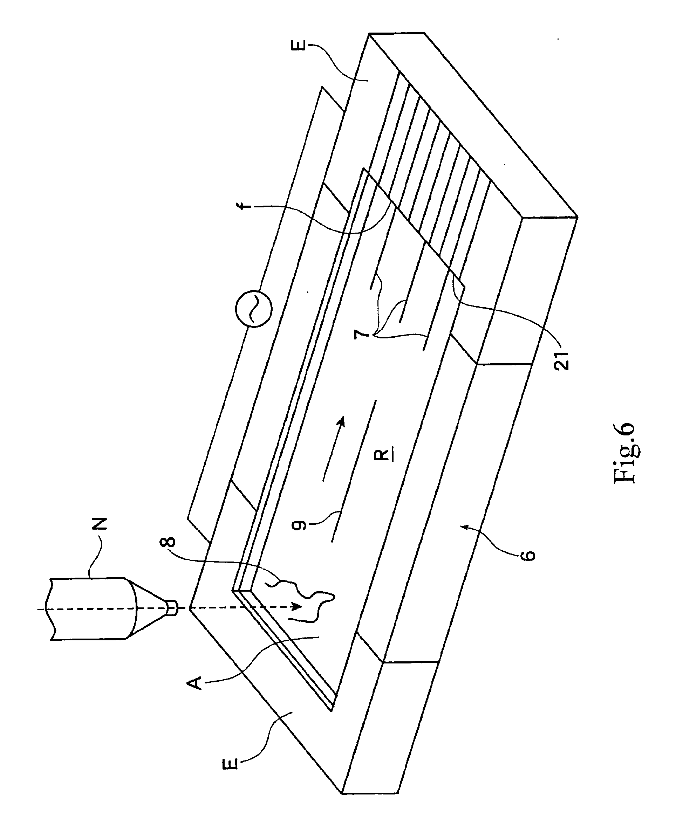

Reference is now made to Fig. 6, which depicts one embodiment of a reaction detecting section equipped with a construction that permits arraying reaction detecting sections on a plate such as a DNA chip. This reaction detecting section 6 is provided with a reaction well A, which is a very small concave region capable of retaining the aqueous solution R, and opposing electrodes E,E arranged facing the reaction well A. Designated at sign 7 in Fig. 6 are a group of single-stranded DNA probes each of which has been immobilized beforehand in an stretched form on a surface f of the electrode E.

Sign 8 in Fig. 6 indicates a single-stranded target nucleic acid added dropwise from a micronozzle N into the reaction well A. Shortly after the dropwise addition, this single-stranded target nucleic acid 8 takes a high-order structure of a random coil form. When a high-frequency ac electric field is applied to the entangled, target single-stranded nucleic acid 8 between the electrodes E-E, the single-stranded nucleic acid can be changed in structure to have an stretched form as designated at sign 9, and moreover, the stretched, single-stranded nucleic acid can be caused to migrate toward the side of the DNA probes 7 along the electric field (electric lines of force).

By providing an off time of application of the electric field after the target, single-stranded nucleic acid 8 has been fully drawn to a region close to the DNA probe 7, hybridization can be allowed to efficiently proceed in a short time by the natural Brownian motion under suitable conditions of pH, temperature and like. Described specifically, between the DNA probe 7 and target, single-stranded nucleic acid 8 both of which are in stretched forms, respectively, high-accuracy hybridization is allowed to proceed with reduced miss-hybridization without being affected by a steric hindrance or the convection of the aqueous solution R when they include mutually-complementary base sequences.

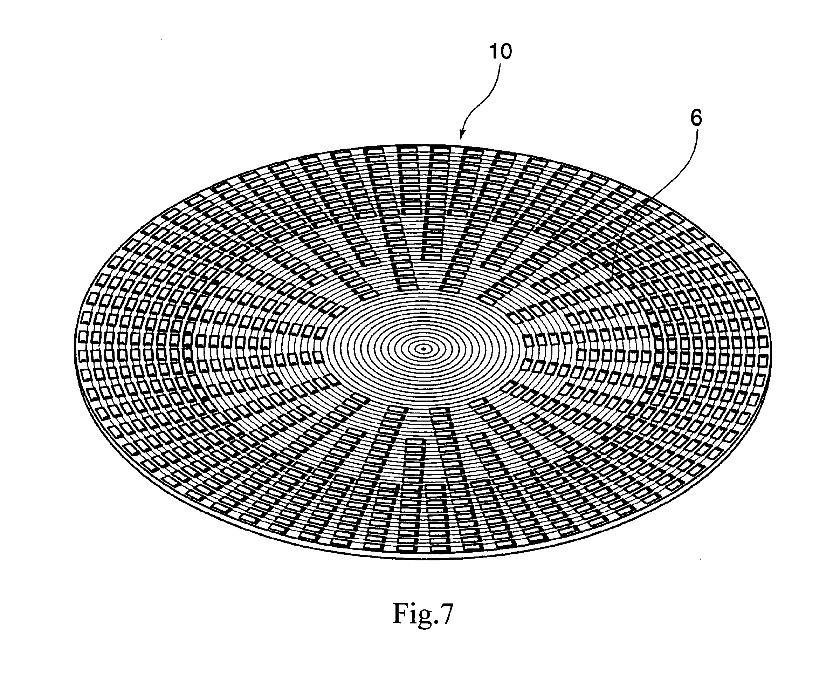

A DNA chip with a number of such reaction detecting sections 6 as illustrated in Fig. 7 arrayed on a substrate can be provided. For example, a number of reaction detection sections 6 as described above are arrayed radially or in the direction of a circumference on such a disk plate 10 as shown in Fig. 7, and desired DNA probes 7 can be immobilized in the reaction detecting sections 6 divided in groups.

### (Example)

An experiment was conducted to verify the stretching action of a high-frequency ac electric field on a single-stranded nucleic acid in accordance with the present invention.

A partial sequence (5 kbp) of λ phage DNA (Takara Shuzo) was amplified by the PCR method (see NAKAYAMA: "Bioexperiments Illustrated", volume 3, Chapter 2, published in Japanese, Shujunsha Co., Ltd. (1998)). Upon conducting the amplification, dTTP and dUTP-FITC were added at a ratio of 1:1 to fluorescently label the PCR product. At the time of the PCR amplification, a primer biotinylated at the 5'end thereof (TOYOBO, LTD.) was used as a one-side primer.

The PCR product was subjected to gel electrophoresis, a band was sliced out by dissection from the position of 5 kbp, and double-stranded DNA fragments were extracted from the gel. "QIAquick Gel Extraction KIT" (QIAGEN K.K.) was used for the extraction of the DNA fragments.

Outline of preparation of single-stranded DNA. In a series of steps, a "DINABEADS Kilobase Binder Kit" (Dynal Inc.) was used. The resultant, double-stranded DNA was caused to bind to avidin applied on the beads, and was then modified with an alkali to liberate the biotin-unmodified, single-stranded DNA of the double-stranded DNA in the liquid phase so that single-stranded DNA was obtained for use in the next step of the experiment.

Described specifically, the beads ("DYNABEADS M-280 streptavidin", 5 µL) in the kit were washed in the binding solution (20 µL), and were then suspended in the binding solution (20 µL). An avidin-biotin reaction was next conducted. Sample DNA (20 µL), which had been amplified by PCR with a biotinylated primer, and the above-described beads suspension, followed by incubation at room temperature for three hours. The beads were collected by a magnet, and subsequent to removal of the supernatant, were washed twice with the washing buffer (40 µL each) to eliminate unreacted DNA.

200 mM sodium hydroxide (20 µL) was added, followed by incubation at 0°C for 10 minutes to effect an alkali modification of DNA. The supernatant with liberated single-stranded DNA contained therein was collected. The supernatant was concentrated using "MICROCON" (Millipore Corporation), and the liquid phase was replaced with ultra pure water to lower the concentration of sodium hydroxide.

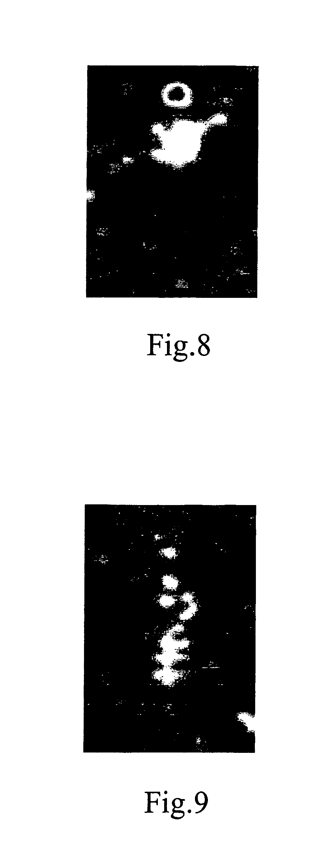

The thus-obtained aqueous DNA solution (5 µL) was placed between opposing aluminum electrodes arranged in a pair with an interval set at 25 µm, and a high-frequency ac electric field voltage of 1 MHz and 1.5 V/µm was applied. As a result, it was possible to have the single strands of DNA stretched in the aqueous solution. That state of stretch was confirmed by observations under an evanescent microscope (manufactured by Olympus Corporation). Microphotographs obtained by those observations are shown in Fig. 8 and Fig. 9.

Fig. 8 is a microphotograph of a single strand of DNA in a random coil form in the aqueous solution before the electric field was applied, and Fig. 9 is a microphotograph of single strands of DNA stretched by the application of the electric field.

### Industrial Applicability

The present invention can be used, for example, in a technology for immobilizing one or more detecting nucleic acids such as detecting DNA probes in stretched forms at predetermined locations on a substrate which makes up a DNA chip or in a technology for performing hybridization while stretching one or more immobilized, detecting nucleic acids and one or more target nucleic acids equipped with complementary strands.

When a high-frequency ac electric field is applied to a single-stranded nucleic acid existing in a free form or in a form immobilized at a terminal thereof in pure water or an aqueous solution of pH 5 to 11, the single-stranded nucleic acid which has been in a random coil form due to thermal motion, can be stretched while avoiding electrolysis. Described specifically, an ion cloud is considered to be formed by phosphoric ions (negative charges), which make up the skeleton of a single-stranded nucleic acid, and their surrounding hydrogen atoms (positive charges) derived from water. Polarization vectors (dipoles) produced by these negative charges and positive charges are, therefore, caused to orient in one direction as a whole upon application of a high-frequency ac electric field, and as a result, the single-stranded nucleic acid can be stretched.

In addition, when a non-uniform electric field is formed in the aqueous solution such that electric lines of force concentrate on a particular position, the single-stranded nucleic acid which exists in a free form in the aqueous solution is caused to move toward the position on which the electric lines of force concentrate. This phenomenon is called "dielectrophoresis". When, with a view to reducing the effect of convection which occurs as a result of an increase in applied voltage, the aqueous solution is placed between opposing electrodes arranged at an interval of 25 µm or shorter and a high-frequency ac electric field is applied to the aqueous solution at a high frequency of 500 kHz or higher and an applied voltage sufficient to give an electric field strength of 1.2 V/µm or higher, for example, dielectric polarization can be surely induced on the single-stranded nucleic acid existing in a random coil form. As a consequence, the single-stranded nucleic acid can be linearly stretched in parallel with the electric field.

By the above-described electrodynamic effect called "dielectrolysis", it is possible to naturally draw the polarized, single-stranded nucleic acid to an edge of the electrode and to immobilize it in a form maintained in contact at one end thereof on the edge of the opposite electrode. This can be used when arranging and immobilizing detecting DNA probes or the like in reaction wells of a DNA chip or the like. The single-stranded nucleic acid stretched in the form immobilized at the one end (terminal) thereof returns into the original random coil form when the electric field is turned off.

With a nucleic acid which is in an stretched form, its base sequent is exposed so that adverse effects due to a steric hindrance or thermal fluctuations can be eliminated. Its hybridization with another nucleic acid having a complementary strand is, therefore, allowed to proceed with high efficiency and high accuracy in a short time.

## Claims

1. A nucleic acid stretch method of stretching the following single-stranded nucleic acid (1) or (2) by causing an ac electric field of a high frequency to act on said single-stranded nucleic acid (1) or (2):
(1) a single-stranded nucleic acid existing in a free form in pure water or an aqueous solution of pH 5 to 11, or
(2) a single-stranded nucleic acid existing in a form immobilized on one or both of opposing electrodes arranged facing said aqueous solution.

2. The nucleic acid stretch method according to claim 1, wherein said high frequency has a frequency of 500 kHz or higher, and a voltage is applied to give an electric field strength of 1.2 V/µm or higher.

3. The nucleic acid stretch method according to claim 1, wherein a distance between said opposing electrodes is set at 40 µm or shorter.

4. The nucleic acid stretch method according to claim 1, wherein said stretch of said single-stranded nucleic acid is effected by dielectrophoresis.

5. A nucleic acid stretch system, **characterized in that** hybridization is conducted by using, as one of complementary strands, a single-stranded nucleic acid stretched by a method according to claim 1.

6. A nucleic acid stretch system provided at least with a reaction well capable of storing an aqueous solution therein and a means for forming a high-frequency ac electric field in said reaction well, **characterized in that** a single-stranded nucleic acid existing in said reaction well is stretched under an action of said high-frequency ac electric field.

7. The nucleic acid stretch system according to claim 6, wherein said reaction well is provided with at least a pair of opposing electrode, and said single-stranded nucleic acid is immobilized at an end thereof on a surface or surfaces of one or both of said opposing electrodes.

8. The nucleic acid stretch system according to claim 7, wherein a distance between said opposing electrodes is 40 µm or shorter.

9. A nucleic acid stretch system, **characterized in that** using an stretched single-stranded nucleic acid as one of complementary strands, hybridization is conducted in said reaction well as described in claim 6.

10. A DNA chip **characterized by** use of a means for stretching a single-stranded nucleic acid, which exists in a free or immobilized form in an aqueous solution of pH 5 to 11, under an action of a high-frequency ac electric field applied to a reaction well with pure water or said aqueous solution retained therein.
